# EUROPÄISCHE PATENTSCHRIFT

(11) **EP 2 654 636 B1**
(45) Veröffentlichungstag und Bekanntmachung des Hinweises auf die Patenterteilung: **05.11.2014**
(21) Anmeldenummer: 11804651.5
(22) Anmeldetag: 22.12.2011
(51) Int. Cl.: A61F 9/008

(54) **VORRICHTUNG ZUR MATERIALBEARBEITUNG EINES WERKSTÜCKS UND VERFAHREN ZUM KALIBRIEREN EINER SOLCHEN VORRICHTUNG**
DEVICE FOR PROCESSING MATERIAL OF A WORKPIECE AND METHOD FOR CALIBRATING SUCH A DEVICE
DISPOSITIF POUR USINER LE MATÉRIAU D'UNE PIÈCE ET PROCÉDÉ POUR ÉTALONNER UN TEL DISPOSITIF

(30) Priorität: 23.12.2010 DE 102010055966
(43) Veröffentlichungstag der Anmeldung: 30.10.2013
(73) Patentinhaber: Rowiak GmbH, 30419 Hannover (DE)
(72) Erfinder: RATHJEN, Christian, 28197 Bremen (DE); LUBATSCHOWSKI, Holger, 30989 Gehrden (DE); RIPKEN, Tammo, 31515 Wunstorf (DE)
(74) Vertreter: Grünecker, Kinkeldey, Stockmair & Schwanhäusser
(86) Internationale Anmeldenummer: PCT/EP2011/006529
(87) Internationale Veröffentlichungsnummer: WO 2012/084255

(56) Entgegenhaltungen:
- WO-A1-2008/112292
- WO-A2-2010/075571
- DE-A1-102009 012 873
- US-A1- 2010 082 017

## Beschreibung

Die vorliegende Erfindung bezieht sich auf eine Vorrichtung zur Materialbearbeitung eines Werkstücks gemäß dem Oberbegriff des Anspruchs 1, sowie auf ein Verfahren zum Kalibrieren einer Vorrichtung zur Materialbearbeitung.

Eine Materialbearbeitungsvorrichtung gemäß dem Oberbegriff des Anspruchs 1 geht aus der DE 10 2006 046 370 A1 hervor. Speziell wird die dort beschriebene Materialbearbeitungsvorrichtung eingesetzt zur Korrektur von Fehlsichtigkeiten durch Augenlaserchirurgie. Bei dieser Augenlaserchirurgie, insbesondere bei der sogenannten Femtosekunden-Lasik, wird ein Auge eines Patienten an ein Kontaktglas angesaugt und fixiert. Anschließend wird gepulste Strahlung eines Femtosekundenlasers in das Innere der Hornhaut des Auges fokussiert, um dort optische Durchbrüche zu erzielen. Am Ort eines optischen Durchbruchs wird das Material durchtrennt. Durch das Scannen des Laserfokus entsteht ein Teppich von aneinander gereihten optischen Durchbrüchen. An diesem Schnitt kann die Hornhaut geöffnet werden, um beispielsweise ein durch die gleiche Technik geschnittenes Lentikel aus dem Inneren der Hornhaut zu entfernen. Auf diese Weise werden die Form der Hornhaut und damit die Brechungseigenschaften des Auges verändert, um die Sehkraft des Patienten wieder zu verbessern.

Wichtig ist bei diesem Verfahren die möglichst exakte Kenntnis über die Lage des Fokus im Werkstück, in diesem Fall also in der Hornhaut des Auges. Abweichungen in der Fokuslage führen zu einer geänderten Schnittgeometrie, die das Behandlungsergebnis deutlich verschlechtern können.

Um die Präzision der Strahlführung zu verbessern, schlägt die DE 10 2006 046 370 A1 vor, die Intensität der Laserstrahlung verringern, bis sie am Ort des Fokus der Laserstrahlung nicht mehr zum Erzeugen eines optischen Durchbruchs ausreicht. Folglich kann der Laser in das Auge eingestrahlt werden, ohne dieses zu bearbeiten. Jetzt wird der Fokus des Lasers auf einer bestimmten Bahn durch das bereits an das Kontaktglas angedockte Auge geführt, beispielsweise auf einer Kreis- oder Spiralbahn. Die vom jeweiligen Ort des Fokus der Laserstrahlung in den Bearbeitungsstrahlengang reflektierte Strahlung wird mittels einer konfokalen Detektoreinheit gemessen. An den Grenzflächen zwischen dem Auge und dem Kontaktglas ist durch den dort vorliegenden Brechungsindexsprung die Reflexion besonders stark. An den Stellen mit besonders hoher Reflexion schneidet also die vorgegebene Bahn des Fokus der Laserstrahlung die Grenzfläche zwischen dem Auge und dem Kontaktglas. Wenn eine Mehrzahl von solchen Schnittpunkten ermittelt wurde, kann unter Kenntnis der Form des Kontaktglases genau auf die Lage des Werkstücks unter dem Kontaktglas zurückgeschlossen werden. Dieses Verfahren erlaubt also eine Kalibrierung der Strahlformungseinheit des Bearbeitungslasers zur Position des Kontaktglases.

Ein anderes Verfahren zur Korrektur der Fokuslage eines Behandlungslasers bei der operativen Fehlsichtigkeitskorrektur eines Auges geht aus der DE 10 2006 053 119 A1 hervor. Dieses Verfahren soll nicht nur die Lage der Hornhaut (Cornea) des Auges an dem Kontaktglas, sondern auch die Verformung der Hornhaut des Auges während der Laserbehandlung berücksichtigen, um die Foki der Bearbeitungslaserstrahlung an die gewünschten Stellen zu platzieren. Die dazu vorgeschlagene Korrektur erfolgt durch ein vergleichsweise kompliziertes, rechnerisches Verfahren.

Nachteilig insbesondere an dem aus der DE 10 2006 046 370 A1 bekannten Verfahren ist, dass es zur Steuerung der Position des Laserfokus lediglich die Grenzfläche zwischen dem Auge und einem Kontaktglas berücksichtig. Dieses Verfahren führt zu Fehlern, wenn tiefere Strukturen im Auge oder in anderen Werkstücken bearbeitet werden sollen und sich dadurch der Abstand zwischen dem Ort der Bearbeitung und der Oberfläche der Probe vergrößert. Ungeeignet ist das bekannte Verfahren beispielsweise für eine Laserkorrektur von Sehfehlern an der natürlichen Augenlinse, die beispielsweise in der DE 10 2008 005 053 A1 beschrieben ist. Darüber hinaus kann das aus der DE 10 2006 046 370 A1 bekannte Verfahren nicht angewendet werden, wenn kein Kontaktglas vorhanden ist.

Die DE 11 2008 002 511 T5 beschreibt ein ophthalmologisches Lasersystem für die Kataraktchirurgie. Um mit dem Chirurgielaser möglichst zielgenau treffen zu können, ist in das Lasersystem ein OCT-Abbildungsmodul integriert. Ein weiteres Lasersystem, das ebenfalls für die Kataraktchirurgie konfiguriert ist und ebenfalls ein OCT-Abbildungsmodul aufweist, ist aus der WO 2008/112292 A1 bekannt. Die DE 10 2009 012 873 A1 beschreibt ein ophthalmologisches Lasersystem für einen anderen chirurgischen Eingriff, nämlich zum Erzeugen von Schnitten in einer Augenlinse oder in der Hornhaut eines Auges.

Aufgabe der vorliegenden Erfindung ist es, eine Vorrichtung und ein Verfahren zur Materialbearbeitung zur Verfügung zu stellen, die mit konstruktiv möglichst einfachen Mitteln eine äußerst präzise, zielgenaue Bearbeitung des Werkstücks ermöglichen.

Diese Aufgabe wird gelöst durch eine Vorrichtung mit den Merkmalen des Anspruchs 1 bzw. durch ein Verfahren mit den Merkmalen des Anspruchs 8. Vorteilhafte Weiterbildungen der Erfindung sind in den Unteransprüchen angegeben.

Im Unterschied zu den herkömmlichen Bearbeitungsvorrichtungen umfasst die erfindungsgemäße Vorrichtung zur Materialbearbeitung eines Werkstücks eine Bildgebungseinheit. Diese Bildgebungseinheit ist dazu eingerichtet, das Werkstück zu untersuchen und insbesondere Strukturen innerhalb des Werkstücks zu erfassen. Bei diesen Strukturen kann es sich um funktionale und um optisch unterscheidbare Komponenten des Werkstücks handeln. Wenn das Werkstück ein menschliches Auge ist, kann es sich bei den erfassten Strukturen beispielsweise um die Augenlinse, die Vorderkammer, die Hornhaut oder andere Komponenten des Auges handeln. Die Bildgebungseinheit bietet den Vorteil, das Werkstück bereits vor der Bearbeitung mit hoher Ortsauflösung untersuchen zu können. Auf diese Weise kann die anschließende Materialbearbeitung vorbereitet werden. Darüber hinaus bietet die Bildgebungseinheit den Vorteil, die Materialbearbeitung im Betrieb der Vorrichtung zu überwachen, zu dokumentieren und gegebenenfalls zu korrigieren.

Die Erfindung geht jedoch über den Einsatz einer Bildgebungseinheit bei der Materialbearbeitung hinaus. Denn sie berücksichtigt, dass physikalische Gründe oder eine nicht völlig exakte Ausrichtung der Bildgebungseinheit mit dem Strahlengang des Bearbeitungslasers zu Abweichungen zwischen dem erwarteten und dem tatsächlich vorliegenden Ort des Fokus der Laserstrahlung im Werkstück führen können. Um diese Abweichungen zu minimieren oder sogar zu eliminieren, ist efindungsgemäß eine Auswerteeinheit vorgesehen und dazu eingerichtet, die mittels der konfokalen Detektoreinheit ermittelte Position des Fokus der Laserstrahlung zu vergleichen mit der erwarteten Lage des Fokus in dem von der Bildgebungseinheit gewonnenen Bild des Werkstücks. Aus diesem Vergleich kann die Lagedifferenz des tatsächlichen und des erwarteten Fokus ermittelt und bei der Bearbeitung des Werkstücks entsprechend Berücksichtigt werden. Dadurch steigt die Präzision der Bearbeitung des Werkstücks erheblich. Die Detektoreinheit ist dabei vorzugsweise eine konfokale Detektoreinheit. Alternativ könnte zur Detektion beispielsweise auch die Second Order Harmonic Generation ausgenutzt werden.

Die erfindungsgemäße Vorrichtung hat dabei den Vorteil, dass kein Kontaktglas vorgesehen sein muss, sodass eine unerwünschte und durch das Kontaktglas hervorgerufene

Deformation des Werkstücks vermieden wird. Im Gegensatz zum Stand der Technik erlaubt die erfindungsgemäße Vorrichtung eine Kalibrierung bzw. einen Abgleich der Koordinatensysteme des Bearbeitungssystems und der Bildgebungseinheit auch in tieferen Lagen im Werkstück, nicht nur an dessen Oberfläche. Auf diese Weise können Fehler vermieden werden, die sich im Stand der Technik aus der Abweichung zwischen, dem Ort der Kalibrierung (d. h. an der Oberfläche des Werkstücks) und dem Ort der Bearbeitung (im Inneren des Werkstücks) ergeben. Über diese Distanz können sich beispielsweise optische Abweichungen ergeben, die bei einer herkömmlichen Kalibrierung zu einer Abweichung der Koordinatensysteme am Ort der Bearbeitung führen - beispielsweise eine Temperaturdrift, die mit herkömmlichen Methoden kaum in den Griff zu bekommen ist. Mit der erfindungsgemäßen Vorrichtung werden solche Fehler vermieden, sodass die Präzision der Bearbeitung weiter gesteigert wird. Ein weiterer Vorteil des erfindungsgemäßen Verfahrens ist, dass das OCT nicht mehr das Kontaktglas mit erfassen muss. Man kann den Messbereich des OCT auf das Zielgebiet einstellen. Dem OCT ist es nun auch erlaubt zu driften. Ein weiterer Vorteil des Verfahrens ergibt sich bei Optiken, die gar nicht mehr auf das Kontaktglas fokussieren können. Systeme mit solchen Optiken können prinzipiell nicht die Verfahren des Standes der Technik benutzen.

Besonders günstig kann es sein, wenn sich die elektromagnetische Strahlung der Bildgebungseinheit in ihrer Wellenlänge von der Laserstrahlung des Bearbeitungslasers unterscheidet. Denn auf diese Weise kann die Strahlung der Bildgebungseinheit weitgehend verlustfrei mit der Bearbeitungslaserstrahlung überlagert werden (beispielsweise durch dichroitische Spiegel) und es kann die jeweils optimale Wellenlänge für die Bearbeitung und für die Bildgebung verwendet werden. Die erfindungsgemäße Vorrichtung bietet dabei den Vorteil, dass sich im Gegensatz zu herkömmlichen Techniken keine dispersionsbedingten Abweichungen der Koordinatensysteme der Bildgebung und der Bearbeitung ergeben, insbesondere bei der Verwendung unterschiedlicher Wellenlängen.

Wie erläutert, kann bei der erfindungsgemäßen Vorrichtung der tatsächliche Ort des Fokus der Bearbeitungslaserstrahlung korreliert oder kalibriert werden mit einem Referenzpunkt im Bildgebungssystem - und zwar auch dann, wenn dieser Referenzpunkt nicht nur an der Oberfläche, sondern im Inneren des Werkstücks liegt. An herkömmlichen Systemen mit einer Kalibrierung an der Grenzfläche zwischen Werkstück und Kontaktglas hingegen konnte die Dispersion im Werkstück zu einer Abweichung zwischen dem erwarteten und dem tatsächlichen Ort des Fokus der Bearbeitungslaserstrahlung führen. Dieses Problem wurde verschärft in komplexen Werkstücken wie beispielsweise menschlichen Augen. Zwar ließen sich die Ortsabweichungen teilweise durch Berücksichtigung einer Dispersionskurve des Materials des Werkstücks wieder herausrechnen. Unsicherheiten in der Dispersionskurve, individuelle Besonderheiten bei der Zusammensetzung des Materials des Werkstücks (z.B. bei Implantaten oder Retina-Chirurgie mit Ersatz-Flüssigkeiten im Augapfel) oder Messfehler sowie technische Unzulänglichkeiten (z.B. eine unvollständige Temperaturkompensation) konnten dennoch zu einem erheblichen Fehler zwischen der beabsichtigten und der tatsächlichen Fokuslage des Bearbeitungslasers führen. Dieser Fehler wird bei der erfndungsgemäßen Vorrichtung durch den im Inneren des Werkstücks erfolgenden Abgleich der Fokuslage des Lasers mit der Bildgebungseinheit vermieden.

Bei der Bildungsbungseinheit handelt es sich vorzugsweise um eine Einheit zur optischen Kohärenztomographie, kurz OCT-Einheit. Sie eignet sich dazu, das Werkstück mit hoher Ortsauflösung zu vermessen und zu charakterisieren. Die Ortsauflösung kann beispielsweise 1 bis 10 Mikrometer betragen. Denkbar wäre alternativ z.B. auch eine Ultraschall-Bildgebungseinheit. Vorzugsweise ist die Steuereinheit dazu konfiguriert, automatisch die Position des Fokus der Laserstrahlung unter Berücksichtigung einer Abweichung der ermittelten Position des Fokus von der erwarteten Lage des Fokus zu korrigieren. Alternativ dazu könnte das Koordinatensystem der Bildgebungseinheit versetzt werden, um die beiden Koordinatensysteme der Bildgebungseinheit und der Bearbeitungseinheit in Deckung zu bringen. Denkbar wäre es, dass einem Bediener auf einer Anzeige ein Vorschlag für eine Korrektur bzw. Kalibrierung der Koordinatensysteme gegeben wird, die der Bediener dann bestätigen kann.

In einer vorteilhaften Ausführungsvariante ist ein dichroitischer Strahlteiler zum Einkoppeln der elektromagnetischen Strahlung der Bildgebungseinheit in den Strahlengang der Laserstrahlung des Bearbeitungslasers vorgesehen. Dieser dichroitische Strahlteiler kann eingesetzt werden, wenn sich die Bearbeitungslaserstrahlung in ihrer Wellenlänge von der Wellenlänge der elektromagnetischen Strahlung der Bildgebungseinheit unterscheidet. Mittels des dichroitischen Strahlteilers können die beiden Strahlungen nahezu verlustfrei in Deckung gebracht und gemeinsam auf das Werkstück gerichtet werden.

Vorzugsweise ist zum Auskoppeln der Laserstrahlung auf die konfokale Detektoreinheit ein Strahlteiler vorgesehen, der mindestens 70 % der Laserstrahlung auf die konfokale Detektoreinheit auskoppelt, vorzugsweise sogar 80 % bis 92 %, idealerweise 90 %. Dies hat zwar den Nachteil, dass ein ebenso großer Anteil der Bearbeitungslaserstrahlung verloren geht. Dieser Nachteil kann jedoch leicht dadurch ausgeglichen werden, dass die Leistung des Bearbeitungslasers entsprechend erhöht wird. Dem steht der enorme Vorteil einer hohen Lichtleistung auf dem konfokalen Detektor entgegen, der zu einer präzisen Messung mit hohem Signal-zu-Rausch-Verhältnis (signal to noise ratio, SNR) führt.

In einer zweckmäßigen Ausführungsvariante weist die konfokale Detektoreinheit eine Blende, eine optische Faser und/oder einen Detektor mit einer vergleichsweise kleinen Detektorfläche auf, beispielsweise mit Abmessungen von 100 Mikrometern oder weniger. Die Blende, der Eingang der optischen Faser oder der Detektor liegen dabei im Fokus einer Optik, die Strahlung aus einem bestimmten Zielgebiet fokussiert, in dem die konfokale Messung durchgeführt wird. Die Blende, die optische Faser oder der Detektor lassen jeweils nur Licht aus diesem vorgegebenen Zielvolumen passieren.

Die Erfindung bezieht sich auch auf ein Verfahren zum Kalibrieren einer Vorrichtung zur Materialbearbeitung eines Werkstücks mit den Merkmalen des Anspruchs 8. Beim erfindungsgemäßen Verfahren wird zur Kalibrierung zunächst die mittlere Leistung des Bearbeitungslasers gegenüber der Bearbeitungsleistung reduziert. Beispielsweise wird die mittlere Leistung auf 30% bis 50% der "Durchbruchsleistung" reduziert, bei der im Material des Werkstücks ein optischer Durchbruch erfolgt. Auf diese Weise wird sichergestellt, dass die reduzierte Leistung des Bearbeitungslasers keine Veränderungen in Material bewirkt. Denkbar wäre es alternativ, den Bearbeitungslaser schwächer zu fokussieren, um auf diese Weise die Intensität am Fokus abzuschwächen. Allerdings könnte diese Variante auf Kosten der Auflösung gehen.

Mit der reduzierten Leistung oder Intensität wird der Bearbeitungslaser in unterschiedliche Tiefen im Inneren des Werkstücks fokussiert. Gleichzeitig wird in einer festen Tiefe z (bei gegebener lateraler Position x, y) im Inneren des Werkstücks eine konfokale Messung der von dort reflektieren Bearbeitungslaserstrahlung durchgeführt. Wenn der Bearbeitungslaser an den Ort der konfokalen Messung fokussiert wird, erreicht das Signal der konfokalen Messung an bestimmten Stellen ein Maximum. Diese Maxima treten an Stellen auf, an dem im Werkstück ein Brechungsindexsprung vorliegt, d. h. wo zwei unterschiedliche Materialbereiche aneinander grenzen. Im erfindungsgemäßen Verfahren wird festgehalten, bei welcher Einstellung der Fokussieroptik für den Bearbeitungslaser die Maxima in der konfokalen Detektion ermittelt wurden. Gleichzeitig oder anschließend erfolgt ein Vergleich mit dem Bild des Werkstücks, das durch die Bildgebungseinheit gewonnen wurde. Auch in diesem Bild können die Brechungsindexsprünge ermittelt werden. Die Kalibrierung der Bildgebungseinheit zum Abgleich mit der ermittelten Tiefe des Fokus des Bearbeitungslasers bei den Maxima des konfokalen Signals erfolgt in Bezug auf einen Referenzpunkt, der im Inneren des Werkstücks und somit in unmittelbarer Nähe des tatsächlichen Bearbeitungsgebiets liegt. Auf diese Weise wird das anschließende Bearbeiten des Materials sehr präzise.

Vorzugsweise werden die Schritte der Fokussierung der Bearbeitungslaserstrahlung mit reduzierter Intensität, der konfokalen Detektion, des Ermittelns der Tiefe des Fokus und des Kalibrierens der Bildgebungseinheit an (bzw. eigentlich unter) mehreren Oberflächenpunkten auf dem Werkstück durchgeführt. Auf diese Weise können Verzerrungen in der Darstellung der Bildgebungseinheit berücksichtigt und ausgeglichen werden.

Günstig ist es, wenn das Kalibrieren der Bildgebungseinheit dynamisch in Abhängigkeit vom jeweiligen Ort des Fokus des Bearbeitungslasers im Werkstück erfolgt. Dadurch können lokale Eigenschaften des Werkstücks bei der Angleichung der Koordinatensysteme berücksichtigt werden.

Denkbar ist es, nicht nur die Bildgebungseinheit an die Fokustiefe der Bearbeitungslaserstrahlung anzupassen, sondern auch die Position des Fokus der Laserstrahlung unter Berücksichtigung einer Abweichung der ermittelten Position des Fokus von der erwarteten Lage des Fokus zu kollimieren. Zu diesem Zweck kann die Fokussieroptik für die Bearbeitungslaserstrahlung entsprechend verstellt werden.

Zur Bildgebung kann zweckmäßigerweise eine optische Kohärenztomographie (OCT) des Werkstücks durchgeführt werden.

Bereits erläutert wurde, dass es sich bei dem Werkstück insbesondere um ein menschliches Auge handeln kann. Besonders vorteilhaft sind die erfindungsgemäße Vorrichtung und das erfindungsgemäße Verfahren zur Vorbereitung einer anschließenden Bearbeitung der Augenlinse oder der Augennetzhaut.

Im Folgenden wird ein vorteilhaftes Ausführungsbeispiel der Erfindung anhand einer Zeichnung näher erläutert.

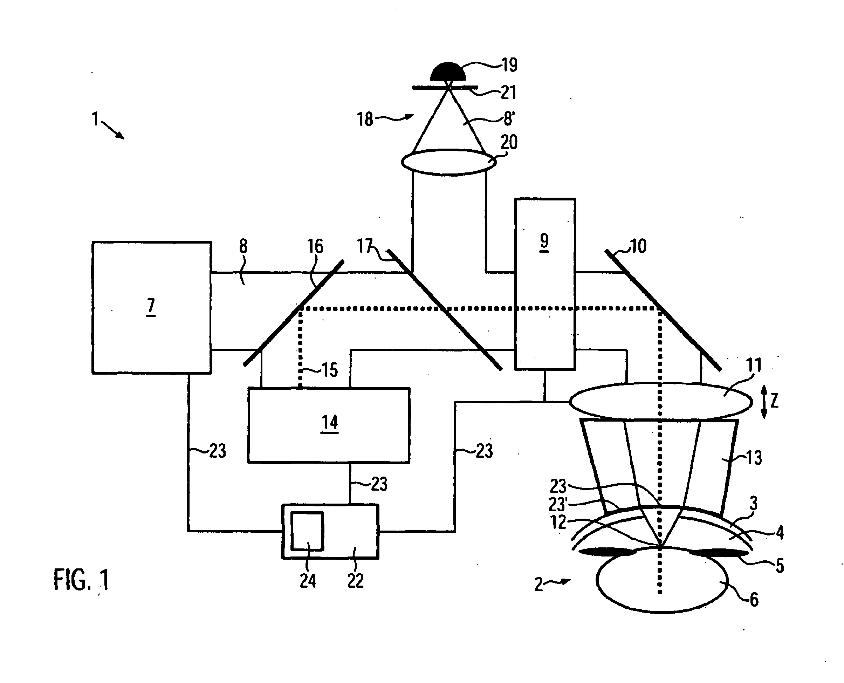

- Figur 1: zeigt in schematischer Ansicht eine erfindungsgemäße Vorrichtung zur Materialbearbeitung eines Werkstücks.

Die in Figur 1 schematisch gezeigte Vorrichtung 1 dient zur Bearbeitung eines Werkstücks 2. Im vorliegenden Ausführungsbeispiel handelt es sich beim Werkstück 2 um ein menschliches Auge. Schematisch dargestellt sind die Hornhaut (Cornea) 3, die Vorderkammer 4, die Iris 5 und die Augenlinse 6.

Die Materialbearbeitungsvorrichtung 1 umfasst einen Bearbeitungslaser 7. Dieser Bearbeitungslaser 7 erzeugt Laserstrahlung 8 in Form von kurzen oder ultrakurzen Laserpulsen. Insbesondere kann es sich beim Bearbeitungslaser 7 um einen Femtosekundenlaser handeln.

Die Laserstrahlung 8 durchläuft eine Strahlformungsoptik 9. In dieser Strahlformungsoptik 9 kann der Laserstrahl beispielsweise aufgeweitet werden, um anschließend einen besonders kleinen Fokus zu erhalten. Zudem kann das Strahlprofil der Laserstrahlung 8 geglättet oder anderweitig moduliert werden.

Die Laserstrahlung 8 läuft ferner über eine Ablenkeinheit 10, bei der es sich um einen verstellbaren, galvanisch angetriebenen Spiegel oder um ein x-y-Paar solcher Ablenkspiegel handelt. Die Ablenkeinheit 10 dient dazu, die Lage des Fokus der Laserstrahlung 8 zu verändern. Zusammen bilden die Strahlformungsoptik 9 und die Ablenkeinheit 10 eine Strahlformungs- und Strahlablenkeinheit.

Ferner durchläuft die Laserstrahlung 8 eine Fokussieroptik 11, beispielsweise eine Fokussierlinse oder ein System von Fokussierlinsen, von denen in Figur 1 nur eine Fokussierlinse dargestellt ist. Die Fokussieroptik 11 bündelt die Laserstrahlung 8 des Bearbeitungslasers 7 zu einem Fokus 12 im Inneren des Werkstücks 2. Im schematisch dargestellten Ausführungsbeispiel liegt der Fokus 12 auf der Vorderseite der Augenlinse 6. Er kann jedoch auch an anderen Stellen im Werkstück 2 liegen, insbesondere im Inneren der Augenlinse 6.

Zwischen der Fokussieroptik 11 und dem Auge 2 befindet sich ein im Ausführungsbeispiel als Kontaktglas 13 ausgebildetes Patienteninterface, an das die Hornhaut 3 des Auges 2 angedockt ist. Das Kontaktglas 13 ist transparent für die Laserstrahlung 8, und es beeinflusst die Qualität des Laserstrahls 8 nicht. Das Patienteninterface kann beispielsweise auch Saugringe umfassen.

Die Materialbearbeitungsvorrichtung 1 verfügt ferner über eine Bildgebungseinheit 14 - im bevorzugten Ausführungsbeispiel eine OCT-Einheit. Die Bildgebungseinheit 14 sendet elektromagnetische Strahlung aus - im Fall einer OCT-Einheit handelt es sich dabei um Laserstrahlung 15. Mit einer gestrichelten Linie ist schematisch der Strahlengang dieser elektromagnetischen Strahlung 15 dargestellt.

Ein dichroitischer Spiegel 16, der sich im Strahlengang der Laserstrahlung 8 des Bearbeitungslasers 7 befindet, transmittiert die Laserstrahlung 8, ist jedoch hochreflektierend für die elektromagnetische Strahlung 15 der OCT-Einheit 14. Der dichroitische Spiegel 16 ist so ausgerichtet, dass er die elektromagnetische Strahlung 15 der OCT-Einheit 14 kollinear in den Strahlengang der Bearbeitungslaserstrahlung 8 einkoppelt. Folglich durchläuft die Strahlung 15 der OCT-Einheit 14 in gleicher Weise wie die Bearbeitungslaserstrahlung 8 die Strahlformungsoptik 9, die Ablenkeinheit 10 und die Fokussieroptik 11, bevor sie in das Werkstück 2 gelangt.

Zwischen dem dichroitischen Spiegel 16 und der Strahlformungsoptik 9 befindet sich im Strahlengang der Bearbeitungslaserstrahlung 8 ein Strahlteiler 17. Dieser Strahlteiler 17 transmittiert bei der Wellenlänge des Bearbeitungslasers 8 etwa 10%, während er die übrigen 90% der Bearbeitungslaserstrahlung 8 reflektiert. Ideal ist es, wenn der Strahlteiler 17 einen möglichst hohen Anteil der elektromagnetischen Strahlung 15 der OCT-Einheit 14 transmittiert.

Der am Fokus von bzw. aus dem Werkstück 2 in den Strahlengang des Bearbeitungslasers 7 zurück reflektierte Anteil 8' der Bearbeitungslaserstrahlung 8 wird vom Strahlteller 17 auf eine zusätzlich zur Bildgebungseinheit 14 vorgesehene, konfokale Detektoreinheit 18 reflektiert. Die konfokale Detektoreinheit 18 umfasst einen Detektor 19, beispielsweise einen CCD-Chip oder einen Photomultiplier (PMT). Eine Linse 20 fokussiert die vom Werkstück 2 reflektierte Bearbeitungslaserstrahlung 8' auf eine Blende 21, die vor dem Detektor 19 angeordnet ist. Alternativ dazu kann ein Detektor 19 mit einer sehr kleinen Detektorfläche verwendet werden, der direkt im Fokus der Linse 20 platziert ist, oder die reflektierte Strahlung 8' kann am Fokus der Linse 20 in eine optische Faser eingekoppelt und über die Faser auf den Detektor 19 geleitet werden.

Die erfindungsgemäße Vorrichtung 1 umfasst ferner eine Steuereinheit 22. Diese Steuereinheit, die beispielsweise in einem PC realisiert sein kann, ist über Steuer- und Datenleitungen 23 mit dem Bearbeitungslaser 7, der aus Strahlformungsoptik 9 und Ablenkeinheit 10 gebildeten Strahlformungseinheit, der Fokussieroptik 11, der OCT-Einheit 14 und der konfokalen Detektoreinheit 18 verbunden. Mittels der Steuerleitungen 23 können Steuerbefehle von der Steuereinheit 22 an den Bearbeitungslaser 7 und die optischen Komponenten im Strahlengang der Laserstrahlung 8 übermittelt werden. Zudem können Messdaten und Statusdaten von diesen optischen Komponenten sowie von der OCT-Einheit 14 und der konfokalen Detektoreinheit 18 an die Steuereinheit 22 übermittelt werden. Zu diesen Daten zählt insbesondere die z-Position der Fokussieroptik 11. Über eine Verstellung in Richtung z, d. h. entlang der optischen Achse der Bearbeitungslaserstrahlung 8, kann die Fokussierlinse 11 die Tiefe des Fokus 12 im Inneren des Werkstücks 2 verändern. Die Dimensionen x und y spannen im Werkstück bzw. Auge 2 eine Ebene senkrecht zur optischen Achse der Bearbeitungslaserstrahlung 8 auf.

Der Betrieb der erfindungsgemäßen Vorrichtung 1 bzw. das erfindungsgemäße Verfahren werden im Folgenden am Beispiel einer Laserbehandlung einer menschlichen Augenlinse 6 beschrieben.

Zunächst wird ein Patient, dessen Auge 2 behandelt werden soll, auf eine Liege gelegt und durch die bewegliche Liege in sich kreuzende Pilotlaserstrahlen oder Spaltlampenbilder in eine vorgegebene Nullposition gefahren. Anschließend überwacht die Bildgebungseinheit 14, im vorliegenden Ausführungsbeispiel also die OCT-Einheit, die Annäherung des Auges 2 an das Kontaktglas 13. Die Unterseite des Kontaktglases 13 und die Vorderseite des Auges 2 sind im OCT erkennbar. Während der Annäherung an das Kontaktglas 13 schaut der Patient auf ein Fixierlicht.

Sobald die Hornhaut 3 des Auges 2 das Kontaktglas 13 berührt, gibt die OCT-Einheit 14 oder die Steuereinheit 22 ein entsprechendes Signal, um die Annäherung abzubrechen. Der Patient schaut, kontrolliert durch visuelle Kontrolle des Arztes, weiter in das Fixationslicht, bis das Auge an dem gekrümmten Kontaktglas 13 durch ein Vakuum fixiert ist. Sobald das Werkstück 2 (im vorliegenden Beispiel das Auge) fixiert ist, vermisst die Bildgebungseinheit 14 das Werkstück 2. Im vorliegenden Beispiel wird das Auge 2 von der OCT-Einheit 14 von der Oberfläche der Cornea 3 bis zur Rückseite der Augenlinse 6 vermessen. Insbesondere wird dabei der Apex 25 (d. h. der vordere Mittelpunkt des Auges 2) der Cornea und/oder der Apex der Augenlinse und/oder vier um den Apex 25 der Cornea oder denjenigen der Augenlinse verteilte, weitere Oberflächenpunkte 25' vermessen. Auf diese Weise erhält die Bildgebungseinheit 14 ein hochauflösendes Bild von den Inneren Strukturen des Auges 2.

Im nächsten Schritt wird die Strahlung 8 des Bearbeitungslasers 7 auf das Auge 2 gestrahlt. Dabei ist die mittlere Leistung des Bearbeitungslasers 7 jedoch auf einen Wert von 50% oder weniger der Durchbruchsleistung eingestellt. Die Durchbruchsleistung ist diejenige Leistung, bei der am Fokus 12 der Laserstrahlung 8 ein optischer Durchbruch im Werkstück 2 erzielt wird. Mittels der Ablenkeinheit 10 wird der Laserstrahl 8 des Bearbeitungslasers 7 auf diejenigen Oberflächenpunkte 23, 23' gerichtet, die zuvor mit der Bildgebungseinheit 14 vermessen wurden. Die Fokussieroptik 11 wird in Richtung z verstellt, sodass der Laserstrahl 8 in z-Richtung am Apex 25 und/oder an den vier weiteren Oberflächenpunkten 25' in unterschiedliche Tiefen im Auge 2 fokussiert wird. Die Stellung der Fokussierlinse 11 in z-Richtung wird über die Datenleitung 23 an die Steuereinheit 22 übermittelt und dort festgehalten.

An jeder Stellung der Fokussieroptik 11 wird die aus dem Zielvolumen, d. h. vom Fokus 12 des Bearbeitungslasers 7, auf die konfokale Detektoreinheit 18 zurück reflektierte Strahlung 8' gemessen. Die Signalstärke der gemessenen Reflexion wird ebenfalls über eine Datenleitung 23 der Steuereinheit 22 zur Verfügung gestellt. Eine Auswerteeinheit 24 in der Steuereinheit 22 vergleicht den Verlauf des Messsignals von der konfokalen Detektoreinheit 18 mit der jeweiligen Stellung der Fokussieroptik 11 in z-Richtung. Jeweils an den Grenzflächen zweier Strukturen mit unterschiedlichen Brechungsindizes im Auge 2 wird ein Maximum des Messsignals von der konfokalen Detektoreinheit 18 festgestellt. Eine Software kann die Maxima durch übliche Algorithmen aus dem Messsignal ermitteln. Diese Maxima werden in der Auswerteeinheit 24 korreliert mit durch die Bildgebungseinheit 14 ermittelten Strukturen im Auge 2. Auf diese Weise erfolgt ein Abgleich zwischen dem Koordinatensystem der Bildgebungseinheit 14 und dem tatsächlichen Ort des Fokus 12 der Laserstrahlung 8 des Bearbeitungslasers 7. Diese Korrelation oder Kalibrierung erfolgt für einen Referenzpunkt im Inneren des Werkstücks 2, unmittelbar am oder im Gebiet, das später durch den Bearbeitungslaser 7 bearbeitet werden soll.

Nachdem die Kalibrierung der Koordinatensysteme der Bildgebungseinheit 14 und der Bearbeitungseinheit, insbesondere der Fokussieroptik 11, erfolgt ist, kann die eigentliche Bearbeitung des Werkstücks 2 stattfinden. Zu diesem Zweck wird die Leistung des Bearbeitungslasers 7 auf die Durchbruchsleistung erhöht, an der am Fokus 12 im Werkstück 2 optische Durchbrüche erzeugt werden. Während der Bearbeitung kann das Zielgebiet, d. h. das im Werkstück 2 bearbeitete Gebiet, mittels der Bildgebungseinheit 14 überwacht werden. Auf diese Weise kann eine Steuerung der Bearbeitung und/oder eine Dokumentation der Bearbeitung erfolgen.

Ausgehend von dem beschriebenen Ausführungsbeispiel können die erfindungsgemäße Vorrichtung 1 und das erfindungsgemäße Verfahren in vielfacher Hinsicht abgewandelt werden. Beispielsweise muss es sich beim Bearbeitungslaser 7 nicht um einen Femtosekundenlaser handeln, sondern es kann sich auch um einen Pikosekundenlaser oder um einen Attosekundenlaser handeln. Möglich ist es auch, einen Abgleich der Koordinatensysteme der Bildgebungseinheit 14 und des Bearbeitungslasers 7 nicht in der Augenlinse 6, sondern auf der Iris 5, auf oder in der Hornhaut 3 oder auf dem Augenhintergrund durchzuführen. Besonders gut eignen sich für die Kalibrierung Flächen oder Strukturen im Werkstück 2, die senkrecht zum Strahlengang des Bearbeitungslasers 7 liegen, da von dort ein besonders hoher Anteil der Strahlung 8' auf die konfokale Detektoreinheit 18 zurück reflektiert wird.

## Patentansprüche

1. Vorrichtung (1) zur Materialbearbeitung eines Werkstücks (2), umfassend:
- einen Bearbeitungslaser (7) zum Erzeugen von Laserstrahlung (8) in Form von kurzen oder ultrakurzen Laserpulsen,
- eine Fokussieroptik (11) zum Fokussieren der Laserstrahlung (8) auf einen Fokus (12) in dem Werkstück (2),
- eine zwischen dem Bearbeitungslaser (7) und dem Werkstück (2) angeordnete Strahlablenkeinheit (10) zum Führen der Laserstrahlung (8) derart, dass die Lage des Fokus (12) der Laserstrahlung (8) in dem Werkstück (2) dreidimensional veränderbar ist,
- eine Steuereinheit (22) zum Ansteuern des Bearbeitungslasers (7), der Fokussieroptik (11) und/oder der Strahlablenkeinheit (10) derart, dass die Lage und/oder die Ausdehnung des Fokus (12) der Laserstrahlung (8) veränderbar ist,
- eine Detektoreinheit (18) zum Detektieren der am Fokus (12) reflektierten oder zurückgestreuten Laserstrahlung (8'),
wobei die Intensität der Laserstrahlung (8) am Fokus auf eine Bearbeitungsintensität und eine demgegenüber niedrigere Messintensität einstellbar ist,
**dadurch gekennzeichnet,**
**dass** die Detektoreinheit (18) eine konfokale Detektoreinheit (18) zum konfokalen Detektieren der am Fokus (12) reflektierten oder zurückgestreuten Laserstrahlung (8') ist, dass eine Bildgebungseinheit (14) unter Verwendung elektromagnetischer Strahlung (15) zum Erfassen von Strukturen innerhalb des Werkstücks (2) vorgesehen ist, wobei die elektromagnetische Strahlung (15) der Bildgebungseinheit (14) über die Strahlablenkeinheit (10) und die Fokussieroptik (11) in das Werkstück (2) einstrahlbar ist,
und **dass** eine Auswerteeinheit (24) vorgesehen und dazu eingerichtet ist, die mittels der Detektoreinheit (18) ermittelte Position des Fokus (12) der Laserstrahlung (8) zu vergleichen mit der erwarteten Lage des Fokus (12) in dem von der Bildgebungseinheit (14) gewonnenen Bild des Werkstücks (2).

2. Vorrichtung nach Anspruch 1, **dadurch gekennzeichnet, dass** sich die elektromagnetische Strahlung (15) der Bildgebungseinheit (14) in ihrer Wellenlänge von der Laserstrahlung (8) des Bearbeitungslasers (7) unterscheidet.

3. Vorrichtung nach einem der vorangehenden Ansprüche, **dadurch gekennzeichnet, dass** die Bildgebungseinheit (14) eine Optische Kohärenztomographieeinheit, OCT-Einheit, ist.

4. Vorrichtung nach einem der vorangehenden Ansprüche, **dadurch gekennzeichnet, dass** die Steuereinheit (22) dazu konfiguriert ist, die Position des Fokus (12) der Laserstrahlung (8) unter Berücksichtigung einer Abweichung der ermittelten Position des Fokus (12) von der erwarteten Lage des Fokus (12) zu korrigieren.

5. Vorrichtung nach einem der vorangehenden Ansprüche, **dadurch gekennzeichnet, dass** ein dichroitischer Strahlteiler (16) vorgesehen ist zum Einkoppeln der elektromagnetischen Strahlung (15) der Bildgebungseinheit (14) in den Strahlengang der Laserstrahlung (8) des Bearbeitungslasers (7).

6. Vorrichtung nach einem der vorangehenden Ansprüche, **dadurch gekennzeichnet, dass** zum Auskoppeln der Laserstrahlung (8') auf die Detektoreinheit (18) ein Strahlteiler (17) vorgesehen ist, der mindestens 70% der Laserstrahlung (8'), vorzugsweise 80% bis 92% der Laserstrahlung (8'), auf die Detektoreinheit (18) auskoppelt.

7. Vorrichtung nach einem der vorangehenden Ansprüche, **dadurch gekennzeichnet, dass** die Detektoreinheit (18) eine Blende (21), eine optische Faser und/oder einen Detektor (19) mit einer Detektorfläche mit Abmessungen von 100 µm oder weniger aufweist.

8. Verfahren zum Kalibrieren einer Vorrichtung (1) zur Materialbearbeitung eines Werkstücks (2), wobei die Vorrichtung (1) einen Bearbeitungslaser (7) zum Bearbeiten des Werkstücks (2) und eine Bildgebungseinheit (14) zum Überwachen oder Dokumentieren der Materialbearbeitung des Werkstücks (2) aufweist, und wobei das Verfahren folgende Schritte aufweist:
a) Fokussieren von Strahlung (8) des Bearbeitungslasers (7) mit gegenüber einer Bearbeitungsintensität reduzierter Intensität in unterschiedliche Tiefen im Inneren des Werkstücks (2) unterhalb eines Oberflächenpunktes (25, 25') auf dem Werkstück (2),
b) konfokales Detektieren der am Fokus (12) der Strahlung des Bearbeitungslasers (7) reflektierten Strahlung (8'),
c) Ermitteln der Tiefe des Fokus (12) bei einem oder mehreren durch Brechungsindexsprünge hervorgerufenen Maxima des bei der Detektion erhaltenen Signals,
d) Kalibrieren der Bildgebungseinheit (14) zum Abgleich mit der ermittelten Tiefe des Fokus (12) des Bearbeitungslasers (7) unter Berücksichtigung der Tiefe des Fokus (12) bei dem mindestens einen Maximum des bei der Detektion erhaltenen Signals.

9. Verfahren nach Anspruch 8, **dadurch gekennzeichnet, dass** die Schritte a) bis d) an mehreren Oberflächenpunkten (25, 25') auf dem Werkstück (2) durchgeführt werden.

10. Verfahren nach einem der Ansprüche 8 oder 9, **dadurch gekennzeichnet, dass** das Kalibrieren der Bildgebungseinheit (14) dynamisch in Abhängigkeit vom jeweiligen Ort des Fokus (12) des Bearbeitungslasers (7) im Werkstück erfolgt.

11. Verfahren nach einem der Ansprüche 8 bis 10, **dadurch gekennzeichnet, dass** die Position des Fokus (12) der Laserstrahlung (8') unter Berücksichtigung einer Abweichung der ermittelten Position des Fokus (12) von der erwarteten Lage des Fokus (12) korrigiert wird.

12. Verfahren nach einem der Ansprüche 8 bis 11, **dadurch gekennzeichnet, dass** die Bildgebungseinheit (14) eine optische Kohärenztomographie (OCT) des Werkstücks (2) durchführt.

## Claims

1. Device (1) for processing material of a work piece (2), comprising:
- a processing laser (7) designed to generate laser radiation (8) in the form of short or ultra-short laser pulses,
- a focusing lens (11) designed to focus the laser radiation (8) to a focal point (12) in the work piece (2),
- a beam deflection unit (10) arranged between the processing laser (7) and the work piece (2), designed to direct the laser radiation (8) in such a way that the position of the focal point (12) of the laser radiation (8) in the work piece (2) is modifiable in three dimensions,
- a control unit (22) designed to control the processing laser (7), the focusing lens (11) and/or the beam deflection unit (10) in such a way that the position and/or the extent of the focal point (12) of the laser radiation (8) is modifiable,
- a detector unit (18) designed to detect the laser radiation (8') reflected or backscattered on the focal point (12),
wherein the intensity of the laser radiation (8) on the focal point is adjustable to a processing intensity and a comparatively lower measuring intensity, **characterised in that**
the detector unit (18) is a confocal detector unit (18) for the confocal detection of the laser radiation (8') reflected or backscattered on the focal point (12), an imaging unit (14) using electromagnetic radiation (15) to record structures within the work piece (2) is provided, wherein the electromagnetic radiation (15) of the imaging unit (14) can shine into the work piece (2) by means of the beam deflection unit (10) and the focusing lens (11),
and that an evaluation unit (24) is provided and equipped to compare the position of the focal point (12) of the laser radiation (8) determined by the detector unit (18) with the expected location of the focal point (12) in the image of the work piece (2) captured by the imaging unit (14).

2. Device according to Claim 1, **characterised in that** the electromagnetic radiation (15) of the imaging unit (14) is distinguished from the laser radiation (8) of the processing laser (7) by its wavelength.

3. Device according to one of the preceding claims, **characterised in that** the imaging unit (14) is an Optical Coherence Tomography (OCT) unit.

4. Device according to one of the preceding claims, **characterised in that** the control unit (22) is configured to rectify the position of the focal point (12) of the laser radiation (8), taking into account any deviation between the ascertained position of the focal point (12) and the expected location of the focal point (12).

5. Device according to one of the preceding claims, **characterised in that** a dichroic beam splitter (16) is provided for coupling the electromagnetic radiation (15) of the imaging unit (14) into the beam path of the laser radiation (8) of the processing laser (7).

6. Device according to one of the preceding claims, **characterised in that** a beam splitter (17) is provided to couple out the laser radiation (8') to the detector unit (18), with said beam splitter (17) coupling out at least 70% of the laser radiation (8'), preferably 80% to 92% of the laser radiation (8'), to the detector unit (18).

7. Device according to one of the preceding claims, **characterised in that** the detector unit (18) features a screen (21), an optical fibre and/or a detector (19) with a detector surface measuring 100 µm or less.

8. A method for calibrating a device (1) for processing material of a work piece (2), wherein the device (1) features a processing laser (7) designed to process the work piece (2) and an imaging unit (14) designed to monitor or document the material processing of the work piece (2), and wherein the method features the following steps:
a) focusing the radiation (8) of the processing laser (7) at different depths within the work piece (2) below a point on the surface (25, 25') on the work piece (2) with a reduced intensity with respect to a processing intensity,
b) confocal detection of the radiation (8') reflected onto the focal point (12) of the radiation of the processing laser (7),
c) ascertaining the depth of the focal point (12) at one or more maxima of the signal obtained at detection caused by refractive index jumps,
d) calibrating the imaging unit (14) for alignment with the ascertained depth of the focal point (12) of the processing laser (7) while taking into account the depth of the focal point (12) at at least one maximum of the signal obtained at detection.

9. Method according to Claim 8, **characterised in that** steps a) to d) are implemented at several surface points (25, 25') on the work piece (2).

10. Method according to one of Claims 8 or 9, **characterised in that** the calibration of the imaging unit (14) takes place dynamically and is subject to the prevailing location of the focal point (12) of the processing laser (7) in the work piece.

11. Method according to one of Claims 8 to 10, **characterised in that** the position of the focal point (12) of the laser radiation (8') is rectified, taking into account any deviation between the confirmed position of the focal point (12) and the expected location of the focal point (12).

12. Method according to one of Claims 8 to 11, **characterised in that** the imaging unit (14) conducts an Optical Coherence Tomography (OCT) of the work piece (2).

## Revendications

1. Dispositif (1) pour le traitement de matière d'une pièce à traiter (2), comprenant :
- un laser de traitement (7) pour produire un rayonnement laser (8) sous la forme d'impulsions laser courtes ou ultra-courtes,
- une optique de focalisation (11) pour focaliser le rayonnement laser (8) en un foyer ou point de focalisation (12) dans la pièce à traiter (2),
- une unité de déviation de rayonnement (10) agencée entre le laser de traitement (7) et la pièce à traiter (2), et destinée au guidage du rayonnement laser (8) de façon à pouvoir faire varier en trois dimensions la position du point de focalisation (12) du rayonnement laser (8) dans la pièce à traiter (2),
- une unité de commande (22) pour commander le laser de traitement (7), l'optique de focalisation (11) et/ou l'unité de déviation de rayonnement (10), de manière à pouvoir faire varier la position et/ou l'étendue du point de focalisation (12) du rayonnement laser (8),
- une unité de détection (18) pour détecter le rayonnement laser (8') réfléchi ou rétrodiffusé au niveau du point de focalisation (12),
l'intensité du rayonnement laser (8) au niveau du point de focalisation pouvant être réglée sur une intensité de traitement et sur une intensité de mesure plus basse que l'intensité de traitement,
**caractérisé**
**en ce que** l'unité de détection (18) est une unité de détection confocale (18) pour la détection confocale du rayonnement laser (8') réfléchi ou rétrodiffusé au niveau du point de focalisation (12),
**en ce qu'**il est prévu une unité d'imagerie (14) utilisant un rayonnement électromagnétique (15) pour la saisie de structures à l'intérieur de la pièce à traiter (2), le rayonnement électromagnétique (15) de l'unité d'imagerie (14) pouvant être envoyé dans la pièce à traiter (2) par l'intermédiaire de l'unité de déviation de rayonnement (10) et de l'optique de focalisation (11),
et **en ce qu'**il est prévu une unité d'évaluation et d'analyse de données (24) conçue pour comparer la position du point de focalisation (12) du rayonnement laser (8), qui a été déterminée au moyen de l'unité de détection (18), à la position attendue du point de focalisation (12) dans l'image de la pièce à traiter (2), obtenue par l'unité d'imagerie (14).

2. Dispositif selon la revendication 1, **caractérisé en ce que** le rayonnement électromagnétique (15) de l'unité d'imagerie (14) se distingue, quant à sa longueur d'onde, du rayonnement laser (8) du laser de traitement (7).

3. Dispositif selon l'une des revendications précédentes, **caractérisé en ce que** l'unité d'imagerie (14) est une unité de tomographie par cohérence optique, à savoir une unité OCT.

4. Dispositif selon l'une des revendications précédentes, **caractérisé en ce que** l'unité de commande (22) est configurée pour corriger la position du point de focalisation (12) du rayonnement laser (8), en tenant compte d'un écart de la position déterminée du point de focalisation (12) par rapport à la position attendue du point de focalisation (12).

5. Dispositif selon l'une des revendications précédentes, **caractérisé en ce qu'**il est prévu une lame séparatrice dichroïque (16) pour injecter le rayonnement électromagnétique (15) de l'unité d'imagerie (14) dans le parcours des rayons du rayonnement laser (8) du laser de traitement (7).

6. Dispositif selon l'une des revendications précédentes, **caractérisé en ce que** pour extraire ou découpler le rayonnement laser (8') vers l'unité de détection (18), il est prévu une lame séparatrice (17), qui extrait ou découple au moins 70% du rayonnement laser (8'), de préférence 80% à 92% du rayonnement laser (8'), vers l'unité de détection (18).

7. Dispositif selon l'une des revendications précédentes, **caractérisé en ce que** l'unité de détection (18) présente un diaphragme (21), une fibre optique et/ou un détecteur (19) avec une surface de détecteur présentant des dimensions de 100 µm ou moins.

8. Procédé pour étalonner un dispositif (1) destiné au traitement de matière d'une pièce à traiter (2), le dispositif (1) comprenant un laser de traitement (7) pour le traitement de la pièce à traiter (2), et une unité d'imagerie (14) pour surveiller ou documenter le traitement de matière de la pièce à traiter (2), le procédé présentant les étapes suivantes :
a) la focalisation de rayonnement (8) du laser de traitement (7) avec une intensité réduite par rapport à l'intensité de traitement, à des profondeurs différentes à l'intérieur de la pièce à traiter (2), en-dessous d'un point de surface (25, 25') de la pièce à traiter (2),
b) la détection confocale du rayonnement (8') réfléchi au niveau du point de focalisation (12) du rayonnement du laser de traitement (7),
c) la détermination de la profondeur du point de focalisation (12) pour un ou plusieurs maximums, produits par des sauts d'indice de réfraction, du signal obtenu lors de la détection,
d) l'étalonnage de l'unité d'imagerie (14) pour l'ajustement avec la profondeur déterminée du point de focalisation (12) du laser de traitement (7), en tenant compte de la profondeur du point de focalisation (12) pour ledit au moins un maximum du signal obtenu lors de la détection.

9. Procédé selon la revendication 8, **caractérisé en ce que** les étapes a) à d) sont effectuées en plusieurs points de surface (25, 25') sur la pièce à traiter (2).

10. Procédé selon l'une des revendications 8 ou 9, **caractérisé en ce que** l'étalonnage de l'unité d'imagerie (14) s'effectue de manière dynamique, en fonction du lieu respectif du point de focalisation (12) du laser de traitement (7) dans la pièce à traiter.

11. Procédé selon l'une des revendications 8 à 10, **caractérisé en ce que** la position du point de focalisation (12) du rayonnement laser (8') est corrigée en tenant compte d'un écart de la position déterminée du point de focalisation (12) par rapport à la position attendue du point de focalisation (12).

12. Procédé selon l'une des revendications 8 à 11, **caractérisé en ce que** l'unité d'imagerie (14) effectue une tomographie par cohérence optique (OCT) de la pièce à traiter (2).
